(19) Europäisches Patentamt
European Patent Office
Office européen des brevets

(11) **EP 3 756 702 B1**

(12) **EUROPÄISCHE PATENTSCHRIFT**

(45) Veröffentlichungstag und Bekanntmachung des Hinweises auf die Patenterteilung:
**30.04.2025 Patentblatt 2025/18**

(21) Anmeldenummer: **19183002.5**

(22) Anmeldetag: **27.06.2019**

(51) Internationale Patentklassifikation (IPC):
***A61M 5/00*** *(2006.01)* ***A61B 50/30*** *(2016.01)*
***A61J 1/10*** *(2006.01)*

(52) Gemeinsame Patentklassifikation (CPC):
**A61M 5/002; A61B 50/30; A61J 1/1468;**
A61B 2050/316

(54) **VERPACKUNG UND VERFAHREN ZUM STERILEN VERPACKEN VON OBJEKTEN FÜR MEDIZINISCHE, PHARMAZEUTISCHE ODER KOSMETISCHE ANWENDUNGEN**

PACKAGE AND METHOD FOR STERILE PACKAGING OF OBJECTS FOR MEDICAL, PHARMACEUTICAL OR COSMETIC APPLICATIONS

EMBALLAGE ET PROCÉDÉ D'EMBALLAGE STÉRILE DES OBJETS POUR APPLICATIONS MÉDICALES, PHARMACEUTIQUES OU COSMÉTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**30.12.2020 Patentblatt 2020/53**

(73) Patentinhaber: SCHOTT Pharma AG & Co. KGaA
**55122 Mainz (DE)**

(72) Erfinder:
• **Wolf, Patrick**
**9000 St. Gallen (CH)**
• **Deutschle, Gregor Fritz**
**65510 Idstein (DE)**
• **Rupertus, Volker**
**55232 Alzey (DE)**

(74) Vertreter: **Fuchs Patentanwälte Partnerschaft mbB**
**Tower 185**
**Friedrich-Ebert-Anlage 35-37**
**60327 Frankfurt am Main (DE)**

(56) Entgegenhaltungen:
**EP-A1- 0 307 173     US-A1- 2006 016 708**

# EP 3 756 702 B1

**Beschreibung**

Die vorliegende Erfindung betrifft eine Verpackung zum sterilen Aufbewahren und Transportieren von Objekten für medizinische, pharmazeutische oder kosmetische Anwendungen, insbesondere von sterilen pharmazeutischen Primärpackmitteln, wie Fläschchen, Ampullen, Spritzen oder Karpulen sowie ein Verfahren zum Verpacken.

Stand der Technik

Pharmazeutische Primärpackmittel, wie Fläschchen, Ampullen, Spritzen, Karpulen und Spritzen, werden von den Herstellern zu den Pharmaunternehmen für die Befüllung in der Regel steril in sogenannten Tubs und Trays verpackt angeliefert. Diese Tubs bestehen zumeist aus einem Träger, welcher eine Vielzahl der Primärpackmittel hält, dem sogenannten Nest, und einer Wanne oder Schale, in welche der Träger eingesetzt wird. Bei den Trays wird eine Vielzahl der Primärpackmittel direkt in das Tray eingesetzt. Üblicherweise werden die Tubs und Trays hierbei mit einem im Flash-Spinning-Verfahren hergestellten Feinstfaservliestoff aus Polyethylen hoher Dichte (HDPE) verschlossen, der unter dem Markennamen Tyvek® der Firma DuPont bekannt ist. Aufgrund des selektiv durchlässigen Tyvek®-Vlieses kann der Tub- bzw. Trayinnenraum auch im verschlossenen Zustand mit Ethylenoxid oder Dampf sterilisiert werden, während eine mikrobielle Barriere geschaffen wird. Vor der Sterilisation werden die Tubs und Trays in der Regel mit ein oder zwei Beuteln dicht umschlossen.

Beim Pharmaunternehmen werden die sterilen Primärpackmittel unter kontrollierten, sterilen Bedingungen, ausgepackt und befüllt. Hierbei werden beim Übergang von höheren in niedriger klassifizierte Reinraumklassen sogenannte Desinfektionsschritte durchgeführt. Bei diesen kann es sich um das Entfernen einer Verpackungsschicht, z. B. eines Beutels, aber auch um eine biologische Dekontamination unter anderem mittels E-Beam, Wasserstoffperoxid oder dem Abwischen mit einer Alkohollösung handeln. Vor dem Überführen des Tubs oder Trays in den aseptischen Bereich, in welchem die Tyvek® Siegelfolie entfernt wird und anschließend das Abfüllen des aktiven Pharmawirkstoffes in die Primärpackmittel stattfindet, findet zunächst noch ein weiterer Dekontaminationsschritt statt. Dies ist notwendig, da die Sterilität der Tub- bzw. Trayaußenseite nicht gewährleistet werden kann.

Beutel werden im Allgemeinen so definiert, dass - abgesehen von der Einfüllöffnung - wenigstens eine der drei Seiten nicht gesiegelt, geschweißt oder geklebt ist, d. h., dass es sich um einen Seitennahtbeutel oder einen Bodennahtbeutel handelt, der aus einem Halbschlauch bzw. einer gefalteten Flachfolie oder einem Vollschlauch hergestellt wurde. Pouches weisen demgegenüber in der allgemeinen Definition auf allen drei Seiten eine Schweiß- oder Siegelnaht oder eine Klebeverbindung auf, da sie aus zwei oder mehreren getrennten Folienbahnen hergestellt werden. Das verschlossene Pouch verfügt demnach dann allseitig über eine Schweiß-, Siegel- oder Klebeverbindung. Im Rahmen dieser Anmeldung wird der Begriff "Beutel" als gemeinschaftlicher Überbegriff sowohl für Beutel als auch Pouches benutzt.

Aus der EP 0 307 173 A1 ist eine sterilisierbare Einwegverpackung für Hospitalutensilien bekannt. Zur Stabilitätsverbesserung und Vermeidung von Faserverschmutzungen auf den verpackten Gegenständen verwendet diese statt der üblichen Papierlagen, die bei einer Dampfsterilisation Schaden nehmen, eine Vlieslage, die dampfpermeabel ist, aber für Bakterien undurchlässig ist. Diese Vlieslage bildet in Kombination mit einer perforierten Kunststofffolienlage als Laminat die eine Seite der Verpackung. Die andere Seite der Verpackung wird von einer einfachen Kunststofffolie oder einem Kunststofffolienlaminat gebildet.

Aus der US 2006/016708 A1 ist ein autoklavierbarer Sterilisationsbeutel bekannt, der aus zwei Kompositfolien-Laminaten eine Pouch formt. Wenigstens das erste Laminat umfasst dabei eine Lage aus einem heißsiegelfähigen transparenten thermoplastischen Polymer als Innenlage und eine Lage aus einem transparenten thermoplastischen Polymer als Außenlage. Dazwischen befindet sich eine ebenfalls transparente Barriereschicht aus molekular orientiertem Polychlorotrifluoroethylen (PCTFE). In Ausführungsformen besteht die Innenlage aus einem Polyolefin und die Außenlage aus Polyethylenterephthalat, Nylon, Polypropylen, Polyethylen oder Cellophan.

Eine Ursache dafür, dass die Sterilität der Beutel-Verpackungen nicht gewährleistet werden kann, liegt in der Dichtheit und Haltbarkeit der Siegelnähte begründet. Sowohl beim Siegeln der Beutel bei der Herstellung als auch beim abschließenden Versiegeln des Beutels nach der Befüllung können diverse Fehler in der Siegelnaht auftreten, die durch eine als Untersiegeln oder Übersiegeln bezeichnete Siegelung außerhalb optimaler Parameter verursacht werden. Bei einem Untersiegeln werden die Lagen mangels ausreichender Temperaturen, Anpressdrücke oder Siegelzeiten nicht genügend verbunden, sodass die sich bildende Siegelnaht nur eine geringe Haftung aufweist. Das auftretende Fehlerbild ist ein Separieren in der Siegelnaht oder Kanal- und Hohlraumbildung.

Beim Übersiegeln werden demgegenüber durch zu hohe Temperaturen, Anpressdrücke oder Siegelzeiten als Pinholes bezeichnete kleine Löcher in der Siegelnaht verursacht, das komplett durchgeschmolzene Polymer seitlich verdrängt, wodurch eine zu dünne Siegelnaht verbleibt, die zudem im Übergangsbereich zur restlichen Folie noch weiter ausgedünnt sein kann, oder im Extremfall sogar das Polymer thermisch geschädigt.

Die Überprüfung der Siegelnähte im laufenden Produktionsprozess ist nur unzureichend möglich, weil bei einer üblichen Kamerainspektion der Siegelnähte von der Folienseite aus die Fehler nur unzureichend zu erkennen sind. Die kristallinen Tyvek® Vliese schmelzen beim Siegeln im Bereich der Siegelnaht auf und verlieren dadurch dort ihre weiße Farbe, wodurch die optische Erkennung der transparenten Siegelnaht und deren Fehlstellen durch den schwachen Kontrast kaum möglich ist.

Aufgabe

Die vorliegende Erfindung soll diese Nachteile des Stands der Technik beheben. Insbesondere soll eine Verpackung zur Verfügung gestellt werden, die zuverlässig und garantierbar die Sterilität des Inhalts gewährleistet, so dass ein zusätzlicher Sterilisierungsschritt beim Pharmaunternehmen nicht mehr notwendig ist.
In einem weiteren Aspekt soll eine verbesserte Detektion von Siegelnahtdefekten ermöglicht werden.

Beschreibung der Erfindung

Gelöst wird diese Aufgabe durch eine Verpackung zum sterilen Verpacken von Objekten für medizinische, pharmazeutische oder kosmetische Anwendungen umfassend wenigstens einen Beutel aus einer ersten und einer zweiten Bahn, die miteinander verbunden sind, die sich dadurch auszeichnet, dass die erste Bahn aus einem selektiv durchlässigen Vliesstoff besteht, die zweite Bahn aus einer kaschierten Folie mit mindestens drei Schichten besteht, wobei die erste Schicht eine auf der Außenseite des Beutels angeordnete Polymerfolie, die zweite Schicht ein Kaschierklebstoff und die dritte Schicht eine auf der Innenseite des Beutels angeordnete Polymerfolie ist, wobei mindestens eine der Schichten der zweiten Bahn ein oder mehrere Pigmente enthält.
In einer Ausführungsform weist das Polymer der ersten Schicht eine Schmelztemperatur auf, die mindestens 20 °C oberhalb der Siegelinitiierungstemperatur der dritten Schicht liegt.
Unter der Schmelztemperatur des Polymers wird im Rahmen dieser Anmeldung die Temperatur des Peakminimums des Schmelzpeaks des zweiten Heizzyklus in einer differentialrasterkalorimetrischen Messung (DSC) mit einer Heizrate von 20 K/min verstanden. Unter der Siegelinitiierungstemperatur des Polymers wird im Rahmen dieser Anmeldung die Temperatur verstanden, bei der bei der Siegelung der beiden Bahnen des Beutels eine nach DIN EN 868-5:2009 Anhang D gemessene Siegelnahtfestigkeit von mindestens 4 N / 15 mm erreicht wird.
In einer Ausführungsform wird die Siegelnaht in einer 8 Bit Graustufenaufnahme, die im Durchlicht aufgenommen und kontrastnormiert wurde, in einem Grauwertbereich von 50 - 200 abgebildet.
Unter kontrastnormiert wird im Rahmen der Anmeldung eine lineare Dehnung der Grauwertskala der Aufnahme auf den vollen Bereich von 0 - 255 der 8 Bit Skala, sodass der Kontrast maximal ist, verstanden. Definieren $g_{min}$ und $g_{max}$ die minimalen und maximalen Grauwerte des Bildes, so berechnet sich die gedehnte Grauwertskala nach der Formel:

$$f(g) = \frac{255}{g_{max} - g_{min}} \cdot (g - g_{min})$$

In einer Ausführungsform weist die Siegelnaht einen in Remission mit 2° Beobachter nach DIN 5033-1:2009-5 gemessenen Transmissionsgrad $\tau_{vis}$ von 10 % - 40 % auf. Der Transmissionsgrad $\tau_{vis}$ kann auch 12 % - 38 %, 14 % - 36 %, 16 % - 34 %, 18 % - 32 %, 20 % - 30 %, 22 % - 28 % oder 24 % - 26 % betragen.
In einer Ausführungsform weisen die in Remission mit 2° Beobachter nach DIN 5033-1:2009-5 gemessenen Transmissionsgrade $\tau_{vis}$ der Siegelnaht und der ersten Bahn (1) eine Differenz von 30 - 75 Prozentpunkten auf. Die Differenz kann auch 35 - 70, 40 - 65, 45 - 60 oder 50 - 55 Prozentpunkte betragen.
In einer Ausführungsform weist die Siegelnaht in einer 8 Bit Graustufenaufnahme, die im Durchlicht aufgenommen und kontrastnormiert wurde, Grauwerte auf, die mindestens 30 Einheiten, 40 Einheiten, 50 Einheiten, 60 Einheiten, 70 Einheiten, 80 Einheiten, 90 Einheiten oder 100 Einheiten oberhalb der Grauwerte des die Siegelnaht umgebenden Bereichs liegen.
Vorzugsweise ist in der zweiten Bahn die erste Schicht eine Polyesterfolie oder Polyamidfolie und die dritte Schicht eine Folie aus Polyethylen-, Polypropylen- oder Polyester-Homo- oder Copolymeren.
Dieser Aufbau des Beutels erzielt gleich mehrere Vorteile. Die Konstruktion der zweiten Bahn als kaschierte Folie verringert die Gefahr von Undichtigkeiten des Beutels aufgrund von Pinholes, die beim Extrudieren der einzelnen Folien auftreten können. Die zwischen den eingesetzten Folienlagen aufgetragene Kaschierkleberschicht sorgt für eine Abdichtung von eventuell vorhandenen Pinholes, sodass Leckagen in der Folie ausgeschlossen werden können.
Eine außenliegende Polyester- oder Polyamidschicht bewirkt eine Verbesserung der Siegelnahtrandbereiche, insbesondere dann, wenn der Wärmeeintrag von der Polyester- bzw. Polyamidseite aus erfolgt. Die außenliegende erste Folienschicht kann hierfür eine Schmelztemperatur aufweisen, die mindestens 20 °C, mindestens 25 °C, mindestens 30 °C, mindestens 35 °C oder mindestens 40 °C oberhalb der Siegelinitiierungstemperatur der innenliegenden dritten Schicht liegt. Die Schmelztemperatur der ersten Folienschicht kann beispielsweise mindestens 200 °C, mindestens 210 °C oder mindestens 220 °C betragen. Somit schmilzt die Außenschicht beim Siegeln nicht mit auf, sondern nur die innere als Siegellage fungierende Schicht schmilzt. Der Übergang der Siegelnaht in den ungesiegelten Bereich des Vliesstoffes wird dadurch wesentlich weniger beansprucht als beispielsweise bei der üblichen einfachen Folienlage aus HDPE, die gegen ein Tyvek® Vlies gesiegelt wird. Hierdurch wird ein Versagen der Tyvek®- bzw. Vliesstofflage an der Siegelnaht vermindert. Überdies kann die Vliesstofflage durch die Siegelnaht komplett aufgeschmolzen werden, was zu einer besseren Verbindung der Lagen mit einer erhöhten Siegelnahtfestigkeit sowie zu einer transparenten oder transluzenten Naht führt. Und nicht zuletzt kann durch den kaschierten Verbund mit der Polyester- oder Polyamidschicht auch noch eine erhöhte Durchstichfestigkeit der Folienseite des Beutels erzielt werden.

Die Einfärbung einer oder mehrerer der Folienschichten sorgt schließlich für eine Verbesserung des Kontrasts und ermöglicht so eine einwandfreie Detektion sowohl von Untersiegelung als auch von Übersiegelung. Aufgrund der transparenten oder transluzenten Siegelnaht kann die Naht im Durchlicht mit der Kamera untersucht werden. Zusammen mit dem zusätzlich erhöhten Kontrast durch die Einfärbung tritt die Siegenaht dann besonders deutlich hervor, wenn die Kamera auf die weiße Vliesstoffseite der Verpackung gerichtet wird. Somit kann in einer Graustufenanalyse des Durchlichtbildes zuverlässig jede Fehlerart in der Siegelnaht detektiert und der Beutel ausgesondert werden. Die minimal detektierbare Fehlstellengröße ist dann nur noch durch die Auflösung des Kamerasystems limitiert.

Die einzusetzenden Pigmente sind nach Menge, Partikelgröße und Farbe dabei erfindungsgemäß so auszuwählen, dass ein für die optische Detektion der Siegelnaht und deren Fehlstellen im Durchlicht oder Auflicht optimales Erscheinungsbild der Siegelnaht entsteht. Dazu wird die Menge, Partikelgröße und Farbe der Pigmente vorzugsweise so eingestellt, dass in einem kontrastnormierten 8 Bit Graustufenbild die Siegelnaht in einem Bereich der Graustufenskala von 50 - 200, 60 - 190, 70 - 180, 80 - 170, 90 - 160, 100 - 150 oder 110 - 140 abgebildet wird. Vorzugsweise betragen die Graustufenwerte < 200, < 190, < 180, < 170, < 160, < 150. Hierdurch lässt sich ein besonders guter Kontrast gegenüber dem umgebenden Bereich aus der nicht gesiegelten Vlieslage erzielen.

Ferner kann insbesondere über die Menge und Partikelgröße der Pigmente auch ein in Remission mit 2° Beobachter nach DIN 5033-1:2009-5 gemessener Transmissionsgrad $\tau_{vis}$ von 10 % - 40 %, 12 % - 38 %, 14 % - 36 %, 16 % - 34 %, 18 % - 32 %, 20 % - 30 %, 22 % - 28 % oder 24 % - 26 % für die Siegelnaht eingestellt werden. Im Zusammenwirken des erhöhten Kontrasts mit der damit erzielten Trübung der Naht, lassen sich die Defektbilder der einzelnen Siegelungsfehler besonders gut detektieren. Ungleichmäßige Mattierungsgrade in der Naht sind ein Zeichen für inhomogenes Aufschmelzverhalten oder nicht korrekten Anpressdruck der Siegelelemente. Kanälchenbildung in der Siegelnaht ist ebenfalls sehr gut zu erkennen, da sich diese klar vom matten Umfeld abheben.

Vorteilhaft kann es auch sein, den in Remission gemessenen Transmissionsgrad $\tau_{vis}$ nicht allein über die Pigmente einzustellen, sondern zusätzlich ein Streuadditiv einzusetzen, das den Anteil an zurückgestreutem Licht und damit den in Remission gemessenen Transmissionsgrad $\tau_{vis}$ erhöht, ohne die Transmission in gleichem Maße zu verringern. Somit verbleibt nach Durchtritt durch die Siegelnaht bei Durchlicht mehr Licht für die Aufnahme, was wiederum dem möglichen Kontrast zu Gute kommt. Solche Streuadditive, die auch selbst eingefärbt sein können, sind dem Fachmann beispielsweise aus der EP 0 269 324 A2 grundsätzlich bekannt. Das Streuadditiv kann dabei in der oder den gleichen Lagen wie das Pigment eingesetzt werden oder separat davon zum Einsatz kommen.

Vorzugsweise ist die Polyester- oder Polyamidfolie der ersten Schicht der zweiten Bahn frei von Pigmenten. Dies bietet den Vorteil, dass das Übersiegeln besonders gut zu detektieren ist. Die transparente oder transluzente Polyester- oder Polyamidschicht schmilzt bei optimalen Siegelbedingungen nicht mit auf. Wenn beim Übersiegeln die darunter liegenden eingefärbten Schichten des Polyethylens und/oder des Kaschierklebers verdrängt werden, ergibt sich im Kamerabild im Durchlicht ein beiderseits von transparenten Bereichen begrenzter eingefärbter Bereich in dem ansonsten weißen Erscheinungsbild des Vliesstoffes.

Der selektiv durchlässige Vliesstoff der ersten Bahn kann ein Feinstfaservliestoff aus Polyethylen hoher Dichte (HDPE), Polypropylen (PP) oder Polyethylenterephthalat (PET) sein. Beispielsweise eigenen sich für die erfindungsgemäßen Zwecke die HDPE Vliesstoffe der Tyvek® Serie von DuPont. Vorzugsweise werden deren ungecoatete Typen eingesetzt (z. B. Tyvek® 1073B). Wesentliche Voraussetzung für die Auswahl eines Vliesstoffes ist dessen Eignung, eine sterilisierbare mikrobielle Barriere bilden zu können, d. h. eine selektive Durchlässigkeit hinsichtlich einer Sperrwirkung für Mikroben bei gleichzeitiger Durchgängigkeit für Sterilisationsmedien wie Ethylenoxid oder Wasserstoffperoxiddämpfe aufzuweisen.

Das Polymer der dritten Schicht der zweiten Bahn kann ein Polyethylen, Polypropylen oder Polyester sein. Die Polymere können dabei als Homopolymere oder als Copolymere, die zu mehr als 50% aus den genannten Monomeren bestehen, eingesetzt werden. Vorzugsweise wird das Polymer dabei entsprechend des Polymers des Vliesstoffs ausgewählt, um die Siegelfähigkeit zu optimieren. Hierbei kann entweder nur die Polymerklasse angeglichen werden oder sogar der gleiche Polymertyp verwendet werden.

Das Polyethylen der dritten Schicht der zweiten Bahn kann ein Polyethylen hoher Dichte (HDPE), Polyethylen mittlerer Dichte (MDPE), Polyethylen niedriger Dichte (LDPE) oder lineares Polyethylen niedriger Dichte (LLDPE) oder deren jeweilige Copolymere, die zu mehr als 50% aus den genannten Monomeren bestehen, sein. Vorzugsweise handelt es sich um HDPE, MDPE oder LDPE. HDPE bietet den Vorteil, dass die Verpackung dann zusätzlich zu Ethylenoxid oder Wasserstoffperoxiddämpfen auch mit Dampf sterilisierbar ist. Eine Sterilisierung mit Strahlung ist bei allen Varianten möglich.

Die Polyesterfolie der ersten Schicht der zweiten Bahn kann aus Polyethylenterephthalat (PET) oder Polybutylenterephthalat (PBT) bestehen. Die Polyamidfolie der ersten Schicht der zweiten Bahn kann aus PA 6, PA 6.6 oder PA 12 bestehen. Mit diesen wird jeweils bei guter Transparenz eine gute Durchstichfestigkeit des Beutels sichergestellt. Sie tragen so in zweifacher Hinsicht zu dem gewünschten Ziel einer garantierbaren Sterilität der Verpackung bei. Einerseits verhindern sie Undichtigkeiten in der Gesamtoberfläche der Verpackung durch Fehlstellen sowie Folienversagen bei mechanischer Beanspruchung und andererseits tragen sie zur Verbesserung der Fehlstellendetektion in den Siegelnähten der Verpackung bei.

Der Kaschierklebstoff der zweiten Schicht der zweiten Bahn ist vorzugsweise ein polyurethanbasierter (PUR) Klebstoff. Insbesondere handelt es sich bei diesem bevorzugt um einen nichtreaktiven PUR Hotmelt Klebstoff. Durch das Kaschieren der beiden Folien wird einerseits bereits ein durchgängiges Loch (Pinhole) in der Gesamtfolie vermieden. Zusätzlich werden eventuell vorhandene Pinholes in den einzelnen Folien durch den PUR basierten Kaschierklebstoff besonders effektiv abgedichtet. Die Auftragsmenge des Kaschierklebstoffs kann 0,5 - 15 $g/m^2$ oder 1,0 - 12 $g/m^2$ oder 1,2 - 10 $g/m^2$ oder 1,4 - 8,0 $g/m^2$ oder 1,6 - 6,0 $g/m^2$ oder 1,8 - 5,0 $g/m^2$ oder 2,0 - 4,0 $g/m^2$ betragen.

In Ausführungsformen der Erfindung erfolgt die Verbindung der Bahnen des Beutels zur Beutelformung durch Versiegeln oder Verkleben und der Verschluss des befüllten Beutels erfolgt mittels einer Siegelnaht. Vorzugsweise erfolgt sowohl die Beutelformung als auch der Verschluss des befüllten Beutels mittels Siegelnähten. Auf diese Weise kann bei dem Beutel allseitig mit Hilfe einer Kamerainspektion der Nähte im Durchlicht die Dichtheit überprüft werden. Zudem ist die Siegelnaht robuster als die Klebeverbindung.

Höchst bevorzugt weisen die Siegelnähte des Beutels eine nach DIN EN 868-5:2009 Anhang D gemessene Siegelnahtfestigkeit von mindestens 20 N / 15 mm, mindestens 22 N / 15 mm, mindestens 25 N / 15 mm oder mindestens 30 N / 15 mm auf.

Die nach DIN EN 14477:2004 bei 100 mm/min Prüfgeschwindigkeit und 0,8 mm Spitzendurchmesser gemessene Durchstoßfestigkeit der ersten Bahn des Beutels beträgt bevorzugt mindestens 10 N, mindestens 11 N, mindestens 12 N, mindestens 13 N oder mindestens 14 N.

Die nach DIN EN 14477:2004 bei 100 mm/min Prüfgeschwindigkeit und 0,8 mm Spitzendurchmesser gemessene Durchstoßfestigkeit der zweiten Bahn des Beutels beträgt bevorzugt mindestens 4,5 N, mindestens 5,0 N, mindestens 5,5 N, mindestens 6,0 N oder mindestens 6,5 N.

In einer Ausführungsform der Erfindung ist innerhalb des Beutels ein Tub in Form einer Wanne oder Schale zur Aufnahme der Objekte angeordnet. Bevorzugt wird in dem Tub ein Träger, ein sogenanntes Nest, angeordnet. Optional ist das Nest fest mit dem Tub verbunden, bevorzugt in einteiliger Bauform. In einer anderen Ausführungsform der Erfindung ist innerhalb des Beutels ein Tray zur Aufnahme der Objekte angeordnet. Eine Vielzahl von pharmazeutischen Primärpackmitteln, wie Fläschchen, Karpulen oder Spritzen, werden so oftmals entweder im Nest oder im Tray aufgenommen und sicher angeordnet.

Höchst bevorzugt ist das Tub oder Tray mit einem selektiv durchlässigen Vliesstoff durch Versiegeln oder Verkleben verschlossen. Dies kann der gleiche Vliesstoff sein, der auch zur Bildung der Beutel verwendet wird. Es kann allerdings auch ein anderer Vliesstoff genutzt werden, der die gleichen Eigenschaften hinsichtlich der selektiven Durchlässigkeit aufweist, aber beispielsweise aus einem Material besteht, das sich besser zur Versieglung mit dem Material des Tubs oder Trays eignet.

In einer weiteren Ausführungsform der Erfindung ist der Beutel von einem oder mehreren weiteren Beuteln umhüllt. Bevorzugt sind die Beutel dabei von identischem Aufbau wie der erste Beutel und bevorzugt lediglich entsprechend größer dimensioniert. Die so generierte Gesamtverpackung ist geeignet in einem schrittweisen Auspackvorgang in immer reinerer Umgebung den Inhalt kontaminationsfrei an den Einsatzort oder Befüllungsort bei einem Pharmaunternehmen zu bringen. Dort wird dementsprechend bei einer Verpackung aus einem versiegelten Tub oder Tray mit zwei Beutelumverpackungen in einer Reinraumumgebung der Klasse C der erste Beutel geöffnet, in Klasse B der zweite Beutel und in Klasse A schließlich das Tub oder Tray.

Ein erfindungsgemäßes Verfahren zum sterilen Verpacken von Objekten für medizinische, pharmazeutische oder kosmetische Anwendungen zeichnet sich dadurch aus, dass eine der vorstehend beschriebenen Verpackungen verwendet wird.

Erfindungsgemäß ist ferner ein Verfahren zur Siegelnahtkontrolle bei einem Beutel aus einer ersten Bahn aus einem selektiv durchlässigen Vliesstoff und einer damit verbundenen zweiten Bahn, die aus einer kaschierten Folie mit mindestens drei Schichten besteht, wobei die erste Schicht eine auf der Außenseite des Beutels angeordnete Polymerfolie, die zweite Schicht ein Kaschierklebstoff und die dritte Schicht eine auf der Innenseite des Beutels angeordnete Polymerfolie ist, wobei das Polymer der ersten Schicht eine Schmelztemperatur aufweist, die mindestens 20 °C oberhalb der Siegelinitiierungstemperatur der dritten Schicht liegt, und wobei mindestens eine der Schichten der zweiten Bahn ein oder mehrere Pigmente enthält, welches die Schritte umfasst

- Bereitstellen der Bahnen des Beutels derart, dass die Siegelnaht in einer 8 Bit Graustufenaufnahme, die im Durchlicht aufgenommen und kontrastnormiert wurde, in einem Grauwertbereich von 50 - 200 abgebildet wird, und die Siegelnaht einen in Remission mit 2° Beobachter nach DIN 5033-1:2009-5 gemessenen Transmissionsgrad $\tau_{vis}$ von 10 % - 40 % aufweist,

- Aufnehmen der Siegelnaht mit einem Kamerasystem im Durchlicht zur Erzeugung einer 8 Bit Graustufenaufnahme und Kontrastnormierung derselben,

- Auswertung der kontrastnormierten 8 Bit Graustufenaufnahme auf Inhomogenitäten in den Grauwerten der Siegelnaht.

Mit dem erfindungsgemäßen Verpackungssystem gelingt es somit, eine optische Kontrolle - bereits mit dem Auge sowie automatisiert mit Hilfe eines Kamerainspektionssystems - zu etablieren, die eine zuverlässige Erkennung von Siegelfehlern sowohl bei Übersiegelung als auch bei Untersiegelung erlaubt. Dementsprechend kann eine Aussortierung von sämtlichen Beuteln mit Fehlstellen in der Siegelnaht erfolgen. Ferner sorgt die mögliche Optimierung der Siegelnaht nicht nur für deren Transparenz oder Transluzenz, die die Detektion verbessert, sondern auch für eine verbesserte Siegelnahtfestigkeit.

Kurzbeschreibung der Figuren

Figur 1 zeigt den Schichtaufbau eines Beutels von Beispiel 1.
Figur 2 zeigt den Schichtaufbau eines Beutels von Beispiel 2.
Figur 3 zeigt ein Auswertungsprofil einer schlechten Siegelnaht von Beispiel 1.
Figur 4 zeigt ein Auswertungsprofil einer guten Siegelnaht von Beispiel 1.

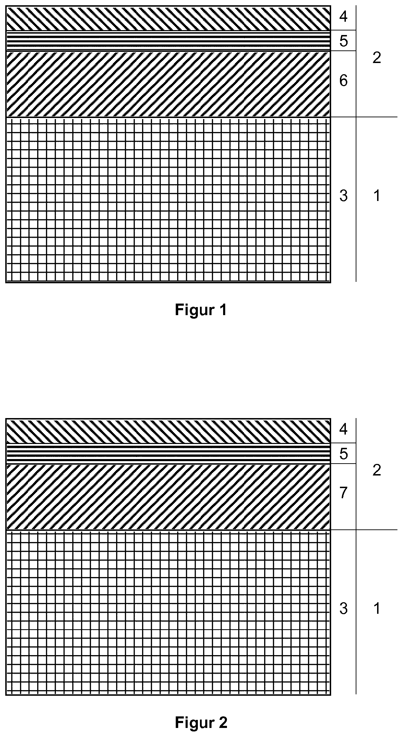

Die Darstellung der Schichtdicken ist in den Figuren 1 und 2 nicht maßstäblich, sondern nur grob angenähert.

Beispiele

Nachfolgend wird die Erfindung anhand von Beispielen dargestellt. Diese dienen lediglich zur exemplarischen Veranschaulichung von erfindungsgemäßen Ausführungsformen und sind nicht als einschränkend zu verstehen.

**Beispiel 1**

Es wurden Beutel aus einer ersten Bahn (1) und einer zweiten Bahn (2) hergestellt. Die erste Bahn (1) bestand aus einem HDPE Vliesstoff (3) des Typs Tyvek® 1073 B uncoated der Firma DuPont mit einer Dicke von ca. 180 $\mu$m. Die zweite Bahn (2) bestand aus einer an der Außenseite angeordneten ersten Schicht (4) aus einer 12 $\mu$m dicken PET Folie und einer damit mittels der zweiten Schicht (5) von 2,5 g/m$^2$ aus einem PUR basierten Hotmelt Kaschierklebstoff verbundenen 50 $\mu$m dicken dritten Schicht (6) aus LDPE. Die dritte Schicht (6) aus LDPE war mit einem Pigment blau eingefärbt, während die anderen beiden Schichten farblos transparent waren. Der Schichtaufbau ist in Fig. 1 abgebildet.

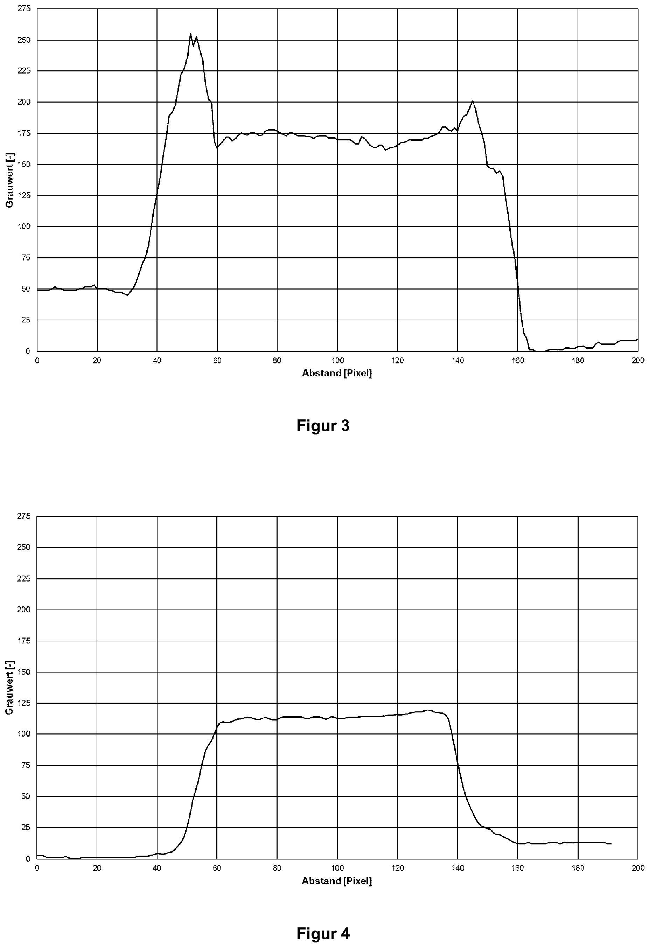

Die Beutelformung erfolgte durch Anbringen von drei Siegelnähten mit einer Breite von 3 mm, 6 mm oder 10 mm mit einem Accu Seal 6300-25-X Impulse Sealer bei 180 - 190 °C, einem Druck von 5,5 - 6 bar und einer Siegelzeit von 3 - 4 s. Der Wärmeeintrag erfolgte einseitig von der Seite der Polyesterfolie aus. Zum Verschließen des befüllten Beutels ist eine weitere Siegelnaht vorgesehen, sodass dann eine allseitig gesiegelte Verpackung vorliegt. Beim Siegeln wurde eine Siegelnaht erzielt, bei der die Vliesstofflage transparent aufgeschmolzen war und sich deutlich die blaue Farbe der LDPE Schicht erkennen ließ. Die Siegelnähte wurden einer Kamerainspektion unterzogen, wobei die Siegelnähte mit Kameras im Durchlicht aufgenommen und die Bilder anschließend analysiert wurden. Hierfür wurden in den normierten Graustufenabbildungen mit einer Auswertungssoftware Profilplots im rechten Winkel über die Siegelnähte erstellt. In Fig. 3 und Fig. 4 ist jeweils beispielhaft ein einzelner Profilschnitt durch die Siegelnaht gezeigt. Aufgetragen wird dabei der Grauskalenwert über die untersuchte Wegstrecke. Für die Beurteilung der Siegelnaht im Produktionsprozess wird von der Software selbstverständlich die Abbildung der gesamten Siegelnaht ausgewertet.

In Fig. 3 ist eine fehlerhafte Naht abgebildet. Deutlich sind die transparenten Stellen an den Kanten der Naht zu sehen, die sich in hellen Peaks zu erkennen geben. Hier ist durch fehlerhafte Temperatur und/oder fehlerhaften Siegelbackendruck die eingefärbte LDPE Siegellage komplett weggeschmolzen, sodass die Ränder nur noch durch die farblos-transparente PET Folie gebildet werden, welche im Durchlicht dann im weißen Bereich der Grauskalenwerte erscheinen.

Demgegenüber zeigt die Fig. 4 eine einwandfreie Siegelnaht, die im Kurvenabbild sehr nahe an der idealen Rechteckkurve liegt.

**Beispiel 2**

Es wurden Beutel aus einer ersten Bahn (1) und einer zweiten Bahn (2) hergestellt. Die erste Bahn (1) bestand aus einem HDPE Vliesstoff (3) des Typs Tyvek® 1073 B uncoated der Firma DuPont mit einer Dicke von ca. 180 $\mu$m. Die zweite Bahn (2) bestand aus einer an der Außenseite angeordneten ersten Schicht (4) aus einer 12 $\mu$m dicken PET Folie und einer damit mittels der zweiten Schicht (5) von 2,5 g/m$^2$ aus einem PUR basierten Kaschierklebstoff verbundenen 50 $\mu$m dicken dritten Schicht (7) aus HDPE. Die dritte Schicht (6) aus HDPE war mit einem Pigment blau eingefärbt, während die anderen beiden Schichten farblos transparent waren. Der Schichtaufbau ist in Fig. 2 abgebildet.

Die Beutelformung erfolgte durch Anbringen von drei Siegelnähten mit einer Breite von 3 mm, 6 mm und 10 mm mit einem Accu Seal 6300-25-X Impulse Sealer bei 180 - 185 °C, einem Druck von 5,7 - 6,2 bar und einer Siegelzeit von 3,5 - 4,5 s. Der Wärmeeintrag erfolgte einseitig von der Seite der Polyesterfolie aus. Zum Verschließen des befüllten Beutels ist eine weitere Siegelnaht vorgesehen, sodass dann eine allseitig gesiegelte Verpackung vorliegt. Beim Siegeln wurde eine Siegelnaht erzielt, bei der die Vliesstofflage transparent aufgeschmolzen war und sich deutlich die blaue Farbe der HDPE Schicht erkennen ließ. Die Siegelnähte wurden wie bei Beispiel 1 einer Kamerainspektion unterzogen, wobei die Siegelnähte mit Kameras im Durchlicht aufgenommen und die Bilder anschließend analysiert wurden. Auch hier waren die Siegelnähte hervorragend zu detektieren und Fehlstellen einwandfrei erkennbar.

**Messmethoden**

Die Siegelnahtfestigkeit wurde nach DIN EN 868-5:2009-09 bestimmt. Beim zu prüfenden Verpackungsmaterial, wurden fünf Streifen mit einer Breite von 15 mm und einer Länge 5 cm ausgeschnitten. Dabei wurde die Probe rechtwinklig zur Siegelnaht ausgeschnitten. Außerdem wurden die Streifen verteilt über die zu prüfende Naht, aber mit einem Mindestabstand von 2 cm zur Außenkante ausgeschnitten. Anschließend wurde auf jedem Streifen, nach der Siegelnaht gegen innen der Verpackung, ein Abstand von 3 cm parallel zur Siegelnaht eingezeichnet. Diese dienten als Einspannmarkierung. Unten wurde das Streifenende vollständig in die untere Zange eingespannt. Die Siegelnaht lag parallel zum oberen Zangenrand. Das andere Ende des Prüflings wurde so in die obere Zange eingespannt, dass die eingezeichneten Markierungen direkt am unteren Rand der oberen Zange zu liegen kamen und der Prüfling noch keiner Spannung ausgesetzt war. Die Prüfung wurde dann mit einer Prüfgeschwindigkeit von 200 mm/min mit ungestütztem Fähnchen durchgeführt, bis ein Versagen des Testmusters zu beobachten war.

Die Durchstoßfestigkeit wurde nach DIN EN 14477:2004 bestimmt. Alle Muster wurden für mindestens 48 h bei einem Klima von 23 °C / 50 % rF gelagert. Anschließend wurden diese in diesem Klima geprüft. Je Probe wurden zehn Einzelmessungen berücksichtigt. Die Folien wurden in die Prüfvorrichtung mit der Innenseite nach oben eingespannt. Der Messfühler von 0,8 mm Durchmesser wurde mit einer Prüfgeschwindigkeit von 100 mm/min bis zum Versagen durch die Folie gestoßen.

Die Messung des in Remission mit 2° Beobachter nach DIN 5033-1:2009-5 gemessenen Transmissionsgrads $\tau_{vis}$ von erfolgte mit einem Spektrometer der Firma Perkin Elmer, Typ Lambda 900, ausgestattet mit einer 60 mm Integrationskugel und einem Probenhalter unter 11°, das im Remissionsmodus eingesetzt wurde und im Bereich von 360 nm bis 780 nm aufzeichnete. Die Beleuchtung erfolgte mit Normlichtart D65. Die Auswertung wurde mit 2° Beobachter nach DIN 5033-1:2009-5 durchgeführt.

**Siegelnahtfestigkeit**

Aus den geformten Beuteln wurden wie vorstehend beschrieben allseitig Probestreifen der Siegelnähte für die Messung der Siegelnahtfestigkeit entnommen. Die in der Tabelle angegebenen Messwerte stellen die dabei jeweils gemessenen Minimalwerte dar.

Die so erhaltenen Probestreifen wurden wie vorstehend beschrieben vermessen. Als Vergleichsbeispiel wurde ein Beutel in der üblichen Ausführungsform mit einer Bahn DuPont Tyvek® 1073B (130-180 $\mu$m) und einer Bahn HDPE (80 $\mu$m) herangezogen. Die Messergebnisse sind in Tabelle 1 aufgeführt.

Tabelle 1

| Muster | Siegelnahtfestigkeit [N / 15 mm] |
|---|---|
| Beispiel 1 | 30 N |
| Beispiel 2 | 27 N |

(fortgesetzt)

| Muster | Siegelnahtfestigkeit [N / 15 mm] |
|---|---|
| Vergleichsbeispiel | 12,5 N |

Die gemessenen Werte zeigen bei den erfindungsgemäßen Beuteln mit einer Laminatbahn im Durchschnitt eine etwa zwei- bis dreimal größere Siegelnahtfestigkeit als bei der gängigen Variante mit Tyvek® 1073B und HDPE.

**Durchstoßfestigkeit**

Aus den Beuteln der Beispiele 1 und 2 sowie der Vergleichsbeispiele 1 und 2 wurden Proben von beiden Bahnen entnommen und gemäß vorstehend beschriebener Methode vermessen. Vergleichsbeispiel 1 entspricht dabei dem Beutel mit einer Bahn DuPont Tyvek® 1073B und einer Bahn HDPE und Vergleichsbeispiel 2 einem Beutel mit einer Bahn aus einem Polypropylen Feinstfaservlies (88-98 g/m$^2$) und einer Bahn aus einem Laminat aus einer Polyesterfolie (12 $\mu$m) und einer Polypropylenfolie (38 $\mu$m). Die Ergebnisse sind in Tabelle 2 zusammengefasst.

Tabelle 2

| Muster | Durchstoßfestigkeit [N] | |
|---|---|---|
| | Bahn 1 | Bahn 2 |
| Beispiel 1 | 16,9 N | 7,3 N |
| Beispiel 2 | 16,9 N | 7,2 N |
| Vergleichsbeispiel 1 | 16,9 N | 3,5 N |
| Vergleichsbeispiel 2 | 16,0 N | 6,8 N |

Die gemessenen Werte zeigen, dass mit dem erfindungsgemäßen Lagenaufbau der Beutel nicht nur eine verbesserte Detektion von Siegelnahtfehlstellen ermöglicht wird, sondern gleichzeitig auch die Verpackung noch robuster wird.

**Transmissionsgrad $\tau_{vis}$**

An Beuteln der Beispiele 1 und 2 wurden jeweils die Siegelnähte und die beiden Bahnen (1) und (2), wie oben beschrieben, in Remission vermessen. Die Proben der Bahnen wurden dabei in einem seitlichen Abstand von 10 mm von der Stelle der untersuchten Siegelnahtstelle genommen.

| | | $\tau_{vis}$ [%] |
|---|---|---|
| Beispiel 1 | Siegelnaht | 18,0 |
| | Bahn (1) | 77,1 |
| | Bahn (2) | 22,1 |
| Beispiel 2 | Siegelnaht | 18,0 |
| | Bahn (1) | 77,5 |
| | Bahn (2) | 22,0 |

**Beispiel 3**

Es wurden mit einem Beutelaufbau wie in Beispiel 1 Fläschchen, die in einem Nest in einer Wanne gelagert wurden, die mit einer Lage Tyvek® 1073 B verschlossen wurde, an einer automatischen Packstraße verpackt. Hierbei wurde die verschlossene Wanne in zwei umschließende Beutel verpackt. Die Versiegelung der Beutel wurde dabei mit dem installierten Kamerainspektionssystem im Durchlicht mit Blickrichtung auf die Tyvek® Seite kontrolliert. Auch hier waren die Siegelnähte hervorragend zu detektieren und Fehlstellen einwandfrei erkennbar.

**Patentansprüche**

1.  Verpackung zum sterilen Verpacken von Objekten für medizinische, pharmazeutische oder kosmetische Anwendungen umfassend wenigstens einen Beutel aus einer ersten und einer zweiten Bahn, die mit einer Siegelnaht miteinander verbunden sind, **dadurch gekennzeichnet, dass**

    - die erste Bahn (1) aus einem selektiv durchlässigen Vliesstoff (3) besteht,
    - die zweite Bahn (2) aus einer kaschierten Folie mit mindestens drei Schichten besteht, wobei die erste Schicht (4) eine auf der Außenseite des Beutels angeordnete Polymerfolie, die zweite Schicht (5) ein Kaschierklebstoff und die dritte Schicht (6, 7) eine auf der Innenseite des Beutels angeordnete Polymerfolie ist, wobei das Polymer der ersten Schicht (4) eine Schmelztemperatur aufweist, die mindestens 20 °C oberhalb der Siegelinitiierungstemperatur der dritten Schicht (6, 7) liegt,
    - mindestens eine der Schichten der zweiten Bahn (2) ein oder mehrere Pigmente enthält,
    - die Siegelnaht in einer 8 Bit Graustufenaufnahme, die im Durchlicht aufgenommen und kontrastnormiert wurde, in einem Grauwertbereich von 50 - 200 abgebildet wird, und
    - die Siegelnaht einen in Remission mit 2° Beobachter nach DIN 5033-1:2009-5 gemessenen Transmissionsgrad $\tau_{vis}$ von 10 % - 40 % aufweist.

2.  Verpackung nach Anspruch 1, **dadurch gekennzeichnet, dass** in der zweiten Bahn (2) die erste Schicht (4) eine Polyesterfolie oder Polyamidfolie und die dritte Schicht (6, 7) eine Folie aus Polyethylen-, Polypropylen- oder Polyester-Homo- oder Copolymeren ist.

3.  Verpackung nach Anspruch 1 oder 2, **dadurch gekennzeichnet, dass** der selektiv durchlässige Vliesstoff (3) der ersten Bahn (1) ein Feinstfaservliestoff aus Polyethylen hoher Dichte (HDPE), Polypropylen (PP) oder Polyethylenterephthalat (PET) ist.

4.  Verpackung nach einem der vorhergehenden Ansprüche, **dadurch gekennzeichnet, dass** das Polyethylen der dritten Schicht (5) der zweiten Bahn (2) ein Polyethylen hoher Dichte (HDPE), Polyethylen mittlerer Dichte (MDPE), Polyethylen niedriger Dichte (LDPE) oder lineares Polyethylen niedriger Dichte (LLDPE) ist.

5.  Verpackung nach einem der vorhergehenden Ansprüche, **dadurch gekennzeichnet, dass** die Polyesterfolie oder Polyamidfolie der ersten Schicht (4) der zweiten Bahn (2) frei von Pigmenten ist.

6.  Verpackung nach einem der vorhergehenden Ansprüche, **dadurch gekennzeichnet, dass** die Polyesterfolie der ersten Schicht (4) der zweiten Bahn (2) aus Polyethylenterephthalat (PET) oder Polybutylenterephthalat (PBT) besteht.

7.  Verpackung nach einem der vorhergehenden Ansprüche, **dadurch gekennzeichnet, dass** die Siegelnähte in einer 8 Bit Graustufenaufnahme, die im Durchlicht aufgenommen und kontrastnormiert wurde, Grauwerte aufweist, die mindestens 30 Einheiten oberhalb der Grauwerte des die Siegelnaht umgebenden Bereichs liegen.

8.  Verpackung nach einem der vorhergehenden Ansprüche, **dadurch gekennzeichnet, dass** die Verbindung der Bahnen (1, 2) des Beutels zur Beutelformung durch Versiegeln oder Verkleben erfolgt und der Verschluss des befüllten Beutels mittels einer Siegelnaht erfolgt.

9.  Verpackung nach einem der vorhergehenden Ansprüche, **dadurch gekennzeichnet, dass** die Siegelnähte des Beutels eine homogene Verfärbung aufweisen.

10. Verpackung nach einem der vorhergehenden Ansprüche, **dadurch gekennzeichnet, dass** die Siegelnähte des Beutels eine nach DIN EN 868-5:2009 Anhang D gemessene Siegelnahtfestigkeit von mindestens 20 N / 15 mm aufweisen.

11. Verpackung nach einem der vorhergehenden Ansprüche, **dadurch gekennzeichnet, dass** die nach DIN EN 14477:2004 bei 100 mm/min Prüfgeschwindigkeit und 0,8 mm Spitzendurchmesser gemessene Durchstoßfestigkeit der ersten Bahn (1) des Beutels mindestens 10 N und/oder der zweiten Bahn (2) des Beutels mindestens 4,5 N beträgt.

12. Verpackung nach einem der vorhergehenden Ansprüche, **dadurch gekennzeichnet, dass** innerhalb des Beutels ein Tub, optional mit einem darin angeordneten Träger, oder ein Tray zur Aufnahme der Objekte angeordnet ist.

13. Verpackung nach Anspruch 12, **dadurch gekennzeichnet, dass** der Tub oder Tray mit einem selektiv durchlässigen Vliesstoff durch Versiegeln oder Verkleben verschlossen ist.

14. Verpackung nach einem der vorhergehenden Ansprüche, **dadurch gekennzeichnet, dass** der Beutel von einem oder mehreren weiteren Beuteln umhüllt ist.

15. Verpackung nach einem der vorhergehenden Ansprüche, **dadurch gekennzeichnet, dass** die in Remission mit 2° Beobachter nach DIN 5033-1:2009-5 gemessenen Transmissionsgrade $\tau_{vis}$ der Siegelnaht und der ersten Bahn (1) eine Differenz von 30 - 75 Prozentpunkten aufweisen.

16. Verfahren zum sterilen Verpacken von Objekten für medizinische, pharmazeutische oder kosmetische Anwendungen, **dadurch gekennzeichnet, dass** eine Verpackung nach einem der Ansprüche 1 bis 15 verwendet wird.

17. Verfahren zur Siegelnahtkontrolle bei einem Beutel aus einer ersten Bahn (1) aus einem selektiv durchlässigen Vliesstoff (3) und einer damit verbundenen zweiten Bahn (2), die aus einer kaschierten Folie mit mindestens drei Schichten besteht, wobei die erste Schicht (4) eine auf der Außenseite des Beutels angeordnete Polymerfolie, die zweite Schicht (5) ein Kaschierklebstoff und die dritte Schicht (6, 7) eine auf der Innenseite des Beutels angeordnete Polymerfolie ist, wobei das Polymer der ersten Schicht (4) eine Schmelztemperatur aufweist, die mindestens 20 °C oberhalb der Siegelinitiierungstemperatur der dritten Schicht (6, 7) liegt, und wobei mindestens eine der Schichten der zweiten Bahn (2) ein oder mehrere Pigmente enthält, umfassend die Schritte

- Bereitstellen der Bahnen des Beutels derart, dass die Siegelnaht in einer 8 Bit Graustufenaufnahme, die im Durchlicht aufgenommen und kontrastnormiert wurde, in einem Grauwertbereich von 50 - 200 abgebildet wird, und die Siegelnaht einen in Remission mit 2° Beobachter nach DIN 5033-1:2009-5 gemessenen Transmissionsgrad $\tau_{vis}$ von 10 % - 40 % aufweist,
- Aufnehmen der Siegelnaht mit einem Kamerasystem im Durchlicht zur Erzeugung einer 8 Bit Graustufenaufnahme und Kontrastnormierung derselben,
- Auswertung der kontrastnormierten 8 Bit Graustufenaufnahme auf Inhomogenitäten in den Grauwerten der Siegelnaht.

**Claims**

1. Package for the sterile packaging of objects for medical, pharmaceutical or cosmetic applications, comprising at least one bag made of a first and a second web that are connected to one another with a sealed seam, **characterized in that**

the first web (1) consists of a selectively permeable non-woven fabric (3);
the second web (2) consists of a laminated film comprising at least three layers, wherein the first layer (4) is a polymer film arranged on the outside of the bag, the second layer (5) is a laminating adhesive and the third layer (6, 7) is a polymer film arranged on the inside of the bag, wherein the polymer of the first layer (4) has a melting temperature which is at least 20°C above the sealing initiation temperature of the third layer (6, 7),
at least one of the layers of the second web (2) includes one or more pigments,
the sealed seam is imaged in a 8-bit greyscale image, which has been taken in transmitted light and contrast-normalized, in a grey-scale value range of 50 - 200, and
the sealed seam has a transmittance $\tau_{vis}$ of 10% - 40% measured in remission with a 2° observer according to DIN 5033-1:2009-5.

2. Package according to claim 1, **characterized in that** in the second web (2) the first layer (4) is a polyester film or a polyamide film and the third layer (6, 7) is a film made of polyethylene, polypropylene or polyester homo- or co-polymers.

3. Package according to claim 1 or 2, **characterized in that** the selectively permeable non-woven fabric (3) of the first web (1) is a microfibrous non-woven fabric made of high-density polyethylene (HDPE), polypropylene (PP) or polyethylene terephthalate (PET).

4. Package according to any one of the preceding claims, **characterized in that** the polyethylene of the third layer (5) of the second web (2) is a high-density polyethylene (HDPE), medium-density polyethylene (MDPE), low-density polyethylene (LDPE) or linear low-density polyethylene (LLDPE).

5. Package according to any of the preceding claims, **characterized in that** the polyester film or polyamide film of the first layer (4) of the second web (2) is free from pigments.

6. Package according to any one of the preceding claims, **characterized in that** the polyester film of the first layer (4) of the second web (2) consists of polyethylene terephthalate (PET) or polybutylene terephthalate (PBT).

7. Package according to any one of the preceding claims, **characterized in that** the sealed seams, in an 8-bit greyscale image taken in transmitted light and contrast-normalized, have grey values which are at least 30 units above the grey values of the area surrounding the sealed seam.

8. Package according to any one of the preceding claims, **characterized in that** the connection of the webs (1, 2) of the bag to form the bag is carried out by sealing or adhesive bonding and the filled bag is closed by means of a sealed seam.

9. Package according to any one of the preceding claims, **characterized in that** the sealed seams of the bag have a homogeneous discoloration.

10. Package according to any one of the preceding claims, **characterized in that** the sealed seams of the bag have a sealed seam strength of at least 20 N/15 mm, measured according to **DIN EN** 868-5:2009, Annex D.

11. Package according to any one of the preceding claims, **characterized in that** the puncture resistance, measured according to DIN EN 14477:2004 at a test speed of 100 mm/min and 0.8 mm tip diameter is at least 10 N for the first web (1) of the bag and at least 4.5 N for the second web (2) of the bag.

12. Package according to any one of the preceding claims, **characterized in that** a tub, optionally comprising a carrier arranged therein, or a tray for receiving the objects is arranged inside the bag.

13. Package according to claim 12, **characterized in that** the tub or tray is closed by sealing or gluing with a selectively permeable non-woven fabric.

14. Package according to any one of the preceding claims, **characterized in that** the bag is wrapped by one or more further bags.

15. Package according to any one of the preceding claims, **characterized in that** the transmittances $\tau_{vis}$ of the sealed seam and of the first web (1), measured in remission with a 2° observer according to DIN 5033-1:2009-5, have a difference of 30 - 75 percentage points.

16. Method for sterile packaging of objects for medical, pharmaceutical or cosmetic applications, **characterized in that** a package according to any one of claims 1 to 15 is used.

17. Method for checking a sealed seam of a bag made from a first web (1) of a selectively permeable nonwoven fabric (3) and a second web (2) connected thereto, which consists of a laminated film comprising at least three layers, wherein the first layer (4) is a polymer film arranged on the outside of the bag, the second layer (5) is a laminating adhesive and the third layer (6, 7) is a polymer film arranged on the inside of the bag, wherein the polymer of the first layer (4) has a melting temperature which is at least 20°C above the seal initiation temperature of the third layer (6, 7), and wherein at least one of the layers of the second web (2) includes one or more pigments, comprising the steps of

providing the webs of the bag in such a way that the sealed seam is imaged in a gray-scale value range of 50 - 200 in an 8-bit grayscale image taken in transmitted light and contrast-normalized, and the sealed seam has a transmittance $\tau_{vis}$ of 10% - 40% as measured in remission with a 2° observer according to DIN 5033-1:2009-5, imaging the sealed seam by use of a camera system in transmitted light to generate an 8-bit grayscale image and contrast normalization of the same,

evaluating the contrast-normalized 8-bit grayscale image for inhomogeneities in the gray values of the sealed seam.

**Revendications**

1.  Emballage pour le conditionnement stérile d'objets destinés à des applications médicales, pharmaceutiques ou cosmétiques, comprenant au moins un sachet constitué d'une première et d'une deuxième bande, qui sont reliées l'une à l'autre par un cordon de soudure, **caractérisé en ce que**

    - la première bande (1) est constituée d'un tissu non tissé (3) sélectivement perméable,
    - la deuxième bande (2) est composée d'un film laminé avec au moins trois couches, dans lequel la première couche (4) comprend un film polymère disposé à la face extérieure du sachet, la deuxième couche (5) est un adhésif de laminage et la troisième couche (6, 7) est un film polymère disposé sur la face intérieure du sachet, le polymère de la première couche (4) ayant une température de fusion qui est supérieure d'au moins 20°C à la température d'initiation du scellage de la troisième couche (6, 7),
    - au moins une des couches de la deuxième bande (2) contient un ou plusieurs pigments,
    - le cordon de soudure est représenté dans une image en niveaux de gris de 8 bits, qui a été prise en lumière transmise et normalisée en contraste, dans une plage de valeurs de gris de 50 à 200, et
    - le cordon de soudure présente une transmittance $\tau$vis de 10 % à 40 %, mesuré en réflexion diffuse avec un observateur de 2° selon la norme **DIN** 5033-1:2009-5.

2.  Emballage selon la revendication 1, **caractérisé en ce que** dans la deuxième bande (2), la première couche (4) est un film de polyester ou un film de polyamide et la troisième couche (6, 7) est un film d'homopolymères ou de copolymères de polyéthylène, de polypropylène ou de polyester.

3.  Emballage selon la revendication 1 ou 2, **caractérisé en ce que** le tissu non tissé (3) sélectivement perméable de la première bande (1) est un tissu non tissé à fibres fines en polyéthylène haute densité (HDPE), en polypropylène (PP) ou en polyéthylène téréphtalate (PET).

4.  Emballage selon l'une des revendications précédentes, **caractérisé en ce que** le polyéthylène de la troisième couche (5) de la deuxième bande (2) est un polyéthylène haute densité (HDPE), un polyéthylène moyenne densité (MDPE), un polyéthylène basse densité (LDPE) ou un polyéthylène basse densité linéaire (LLDPE).

5.  Emballage selon l'une des revendications précédentes, **caractérisé en ce que** le film polyester ou le film polyamide de la première couche (4) de la deuxième bande (2) est exempt de pigments.

6.  Emballage selon l'une des revendications précédentes, **caractérisé en ce que** le film polyester de la première couche (4) de la deuxième bande (2) est en polyéthylène téréphtalate (PET) ou en polybutylène téréphtalate (PBT).

7.  Emballage selon l'une des revendications précédentes, **caractérisé en ce que** les joints de scellage présentent, dans une photographie en niveaux de gris de 8 bits prise en lumière transmise et normalisée en contraste, des valeurs de gris qui sont supérieures d'au moins 30 unités aux valeurs de gris de la zone entourant le cordon de soudure.

8.  Emballage selon l'une des revendications précédentes, **caractérisé en ce que** la liaison des bandes (1, 2) du sachet pour former le sachet est réalisée par scellage ou collage et la fermeture du sachet rempli est réalisée au moyen d'un cordon de soudure.

9.  Emballage selon l'une des revendications précédentes, **caractérisé en ce que** les cordons de soudures du sachet présentent une coloration homogène.

10. Emballage selon l'une des revendications précédentes, **caractérisé en ce que** les cordons de soudure du sachet présentent une résistance de soudure mesurée selon la norme DIN EN 868-5:2009 annexe D d'au moins 20 N / 15 mm.

11. Emballage selon l'une des revendications précédentes, **caractérisé en ce que** la résistance à la perforation mesurée selon la norme DIN EN 14477:2004 à une vitesse de test de 100 mm/min et un diamètre de pointe de 0,8 mm est d'au moins 10 N pour la première bande (1) du sachet et/ou d'au moins 4,5 N pour la deuxième bande (2) du sachet.

12. Emballage selon l'une des revendications précédentes, **caractérisé en ce qu'**un bac, éventuellement avec un support disposé à l'intérieur, ou un plateau pour recevoir les objets est disposé à l'intérieur du sachet.

13. Emballage selon la revendication 12, **caractérisé en ce que** le bac ou le plateau est fermé par un non-tissé sélectivement perméable par scellage ou collage.

14. Emballage selon l'une des revendications précédentes, **caractérisé en ce que** le sachet est enveloppé par un ou plusieurs autres sachets.

15. Emballage selon l'une des revendications précédentes, **caractérisé en ce que** les coefficients de transmission $\tau$vis du cordon de soudure et de la première bande (1), mesurés en réflexion diffuse avec un observateur de 2° selon la norme DIN 5033-1:2009-5, présentent une différence de 30 à 75 points de pourcentage.

16. Procédé d'emballage stérile d'objets destinés à des applications médicales, pharmaceutiques ou cosmétiques, **caractérisé en ce que** l'on utilise un emballage selon l'une des revendications 1 à 15.

17. Procédé de contrôle de la soudure d'un sachet constitué d'une première bande (1) d'un tissu non-tissé (3) sélectivement perméable et d'une deuxième bande (2) reliée à la première et constituée d'un film laminé comprenant au moins trois couches, dans lequel la première couche (4) est un film polymère disposé sur la face extérieure du sachet, la deuxième couche (5) étant un adhésif de laminage et la troisième couche (6, 7) est un film polymère disposé sur la face intérieure du sachet, le polymère de la première couche (4) ayant une température de fusion qui est supérieure d'au moins 20°C à la température d'amorçage du scellage de la troisième couche (6, 7), et au moins l'une des couches de la deuxième bande (2) contenant un ou plusieurs pigments, comprenant les étapes consistant à

   - Préparer des bandes du sachet de telle sorte que le la soudure soit représentée dans une image en niveaux de gris de 8 bits, qui a été prise en lumière transmise et normalisée en contraste, dans une plage de niveaux de gris de 50 à 200, et que le cordon de soudure présente un degré de transmission $\tau$vis de 10 % à 40 %, mesuré en réflexion diffuse avec un observateur de 2° selon la norme **DIN** 5033-1:2009-5,
   - Prendre une image du cordon de soudure avec un système de caméra en lumière transmise pour générer une image en niveaux de gris 8 bits et une normalisation du contraste de celle-ci,
   - Evaluer l'image en niveaux de gris de 8 bits normalisé en contraste pour détecter des inhomogénéités dans les valeurs de gris du cordon de soudure.

**Figur 1**

**Figur 2**

**Figur 3**

**Figur 4**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0307173 A1
- US 2006016708 A1
- EP 0269324 A2